(19) Europäisches Patentamt
European Patent Office
Office européen des brevets

(11) EP 1 028 993 B1

# (12) EUROPÄISCHE PATENTSCHRIFT

(45) Veröffentlichungstag und Bekanntmachung des Hinweises auf die Patenterteilung:
**19.03.2003 Patentblatt 2003/12**

(21) Anmeldenummer: **98959845.3**

(22) Anmeldetag: **04.11.1998**

(51) Int Cl.[7]: **C08G 77/38**

(86) Internationale Anmeldenummer:
**PCT/EP98/07047**

(87) Internationale Veröffentlichungsnummer:
**WO 99/023137 (14.05.1999 Gazette 1999/19)**

## (54) OBERFLÄCHLICH HYDROPHILIERTE SILIKONELASTOMERE, VERFAHREN ZU DEREN HERSTELLUNG SOWIE DEREN VERWENDUNG

SUPERFICIAL HYDROPHILIZED SILICON ELASTOMERS, A METHOD FOR THE PRODUCTION THEREOF AND THEIR APPLICATION

ELASTOMERES SILICONES HYDROPHILISES EN SURFACE, LEURS PROCEDES DE PRODUCTION ET LEUR UTILISATION

(84) Benannte Vertragsstaaten:
**AT BE CH CY DE DK ES FI FR GB GR IE IT LI LU MC NL PT SE**

(30) Priorität: **04.11.1997 DE 19748606**

(43) Veröffentlichungstag der Anmeldung:
**23.08.2000 Patentblatt 2000/34**

(73) Patentinhaber: **GE Bayer Silicones GmbH & Co. KG**
**40699 Erkrath (DE)**

(72) Erfinder:
- **STEINBERGER, Helmut**
  **D-51375 Leverkusen (DE)**
- **WAGNER, Roland**
  **D-81549 München (DE)**
- **MALLWITZ, Frank**
  **D-12683 Berlin (DE)**
- **WU, Yu Ling**
  **D-13347 Berlin (DE)**
- **STELZLE, Martin**
  **D-72766 Reutlingen (DE)**

(56) Entgegenhaltungen:
**EP-A- 0 671 163** **EP-A- 0 882 753**

- **JAMES H. SILVER ET AL.: "Surface and blood-contacting properties of alkylsiloxane monolayers supported on silicone rubber" JOURNAL OF BIOMEDICAL MATERIALS RESEARCH, Bd. 29, 1995, Seiten 535-548, XP002096240 WILEY, NEW YORK, NY; US in der Anmeldung erwähnt**
- **DATABASE WPI Week 7735 Derwent Publications Ltd., London, GB; AN 77-61710y XP002096241 & JP 52 084258 A (TOYO CONTACT LENS CO. LTD.), 13. Juli 1977**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft selektiv oberflächlich hydrophilierte Silikonelastomere, Verfahren zu deren Herstellung sowie deren Verwendung. Vernetzte Polydimethylsiloxane (PDMS) sind schwach polare, niederenergetische und hydrophobe Materialien (Randwinkel von Wasser >90°).
Zur Modifizierung u.a. der grenzflächenenergetischen Eigenschaften werden deshalb standardmäßig andere organische Substituenten, wie z.B. Phenyl- und Trifluoropropylgruppen am Polymerrückgrat verankert, wobei jedoch gleichzeitig eine nicht immer erwünschte Modifizierung des Silicons (Bulkeigenschaften) auftritt.
In EP-A 398 745 wurden zur Hydrophilierung der Oberfläche SiH und gleichzeitig oligooxyethylenfunktionalisierte Siloxane in die zu vernetzende Mischung eingebracht. Diese Hydrophilierung war jedoch nachteiligerweise von einer Verschlechterung der Bulkeigenschaften begleitet.
Es sind aus diesem Grund heraus andere Wege zur Einstellung der grenzflächenenergetischen Eigenschaften vorgeschlagen worden. So wurden gemäß Colloids und Surfaces, 1986, 20, 277, grenzflächenaktive, alkylarymodifizierte Polyethylenoxidderivate mit PDMS compoundiert. Zwar kann auf diese Weise ebenfalls wirksam die Oberflächenenergie eingestellt werden, Probleme durch die gleichzeitige Beeinflussung der Bulkeigenschaften (Phasenseparation und Ausspüleffekte) können aber nur schwer beherrscht werden.
Eine Oberflächenhydrophilierung durch Kohlehydratderivate ist aus EP-A 317 377 bekannt. Dem Wesen nach werden a) Vinylsiloxane, b) H-Siloxane sowie c) SH und gleichzeitig epoxyfunktionalisierte Siloxane miteinander vernetzt und in einer anschließenden Reaktion an der Oberfläche befindliche Epoxygruppen mit Saccharidderivaten, bevorzugt Gluconsäure umgesetzt. Zwar erfolgt gemäß diesem Konzept die Hydrophilierung oberflächenselektiv, jedoch verbleibt der überwiegende Anteil der latent reaktiven Epoxygruppen im Bulk und beeinflußt in unerwünschter Form dessen Eigenschaften.
Die häufig angewandte Sauerstoffplasmabehandlung stellt eine effiziente Methode zur selektiven Oberflächenmodifizierung von PDMS dar. Allerdings ermöglichen die Bewegungen der Polymerkettensegmente den generierten Hydroxylgruppen, im Austausch gegen Methylgruppen in das Polymerinnere zu wandern, da hierdurch eine thermodynamisch begünstigte Minimierung der Oberflächenenergie erfolgen kann (I. Noda, Chemisty & Industry, 21.10.1991, S. 749).
Eine Modifizierung dieser temporär hydroxylierten Oberflächen durch Gasphasensilylierung mit Trichlorsilanderivaten führt gemäß Macromolecules 1993, 26, 5870 zu Alkyl-, Alkenyl-, Perfluoroalkyl- und Carboxylatderivaten. Nachteilig an dieser Verfahrensweise ist jedoch die Beschränkung auf verdampfbare Chlorsilane.
In einem Fall ist die Modifizierung einer solchen Oberfläche in Flüssigphase mit einem Trichlorsilanderivat, welches über drei Ethylenoxideinheiten und eine Acetatendgruppe verfügt, ausgeführt worden (siehe J. Biomed. Mat. Research 1995, 29, 535). Dieses hat jedoch den Nachteil, daß aufgrund der zu kurzen Kette noch keine gute Blutverträglichkeit gegeben ist.
Keine dieser Lösungen erfüllt jedoch die Forderungen nach selektiver Modifizierbarkeit der Oberfläche mit geringer Eindringtiefe, sehr hoher Variabilität hinsichtlich der chemischen und energetischen Natur der Oberflächenmodifizierung und gesicherter Langzeitstabilität des erreichten Oberflächenzustandes.
Aufgabe der Erfindung war daher die Bereitstellung von oberflächlich hydrophilierten Silikonelastomeren, die die im Stand der Technik bekannten Nachteile nicht aufweisen.
JP-A-52 084258 und die nichtvorveröffentlichte EP-A-0882753 offenbaren hydrophile Gruppen aufweisende Silikonelastomere.
Überraschenderweise wurde nun gefunden, daß Silikonelastomere, deren in der Oberfläche sitzende Siliciumatome zu 1 bis 100 % in Form von Einheiten der Formel

$$[Z\ X\ Y\ SiO_{1/2}] \qquad (I)$$

vorliegen, das geforderte Eigenschaftsprofil aufweisen und sich daraus langzeitstabile hydrophile Elastomere herstellen lassen.
Gegenstand der Erfindung sind daher selektiv oberflächlich hydrophilierte Silikonelastomere wie im Anspruch definiert.,
**[0002]** Der Begriff heteroelementsubstituiert umfaßt dabei als Element:, F, N, O.
In einer bevorzugten Ausführungsform der Erfindung haben die Reste $R^1$ in den erfindungsgemäßen oberflächlich hydrophilierten Silikonelastomeren die folgende Bedeutung:

R1 = $-CH_2OH$,
$-CH_2(OCH_2CH_2)_m(OCH_2CH[CH_3])_n(OCH_2CH[CH_2OH))_oOE$,
$-CH_2OC(O)CH_2CH_2COOH$, $-CH_2OC(O)CH_2CH_2COO^-$,

$-CH_2OC(O)CH-CHCOOH$, $-CH_2OC(O)CH=CHCOO^-$, $-(CH_2)_pCH(OH)CH_2R^2$, $-(CH2)_pOCH_2CH(OH)CH_2R^2$, $-(CH_2)_qR^3$, $-(CH_2)_pO(CH_2)_pR^3$,

mit

- m = 0 bis 100, bevorzugt 0 bis 50, besonders bevorzugt 5 bis 50, ganz besonders bevorzugt 7 bis 20,
- n = 0 bis 60, bevorzugt 0 bis 30, besonders bevorzugt 0 bis 10, ganz besonders bevorzugt 1 bis 10,
- o = 0 bis 60, bevorzugt 0 bis 50, besonders bevorzugt 5 bis 50, ganz besonders bevorzugt 5 bis 20, mit $0 \leq m+n+o \leq 100$, bevorzugt $0 \leq m+n+o \leq 50$, besonders bevorzugt $5 \leq m+n+o \leq 50$, ganz besonders bevorzugt $7 \leq m+n+o \leq 50$,
- p = 1 bis 10, bevorzugt 2 bis 10, besonders bevorzugt 2 bis 4,
- q = 0 bis 5, bevorzugt 0 bis 1, besonders bevorzugt 0,
- E = $CH_3$, H, $C_4H_9$, t- $C_4H_9$
- $R^2$ = $-NH[(CH_2)_2NH]_sR^4$, $-NH[(CH_2)_3NH]_sR^4$, $-NHCH_2CH(CH_3)NHR^4$, $N[(CH_2)_2NHR^4]_2$, $-N[(CH_2)_3NHR^4]_2$,

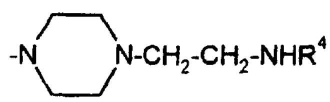

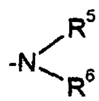

- $R^3$ = $-(CF_2)_tCF_3$, $-(CF_2)_tCF_2H$

mit

- s = 0 bis 10, bevorzugt 1 bis 5, besonders bevorzugt 1 bis 2, ganz besonders bevorzugt 1,
- t = 0 bis 19, bevorzugt 3 bis 19, besonders bevorzugt 5 bis 19, ganz besonders bevorzugt 5 bis 15 und 10 bis 19,
- $R^4$ = H, $C_1$-$C_{12}$ geradkettiger und/oder cyclischer mono- oder polyhydroxylierter Kohlenwassersstoffrest, der sich bevorzugt von Hydroxycarbonsäuren, Sacchariden, bevorzugt Mono- und Disacchariden, besonders bevorzugt oxydierten Mono- und Disacchariden der Formel

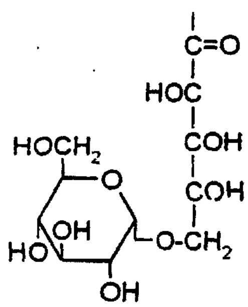

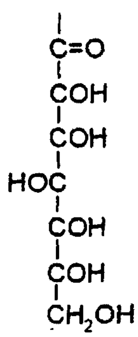

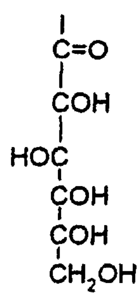

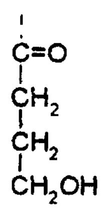

- $R^5$ = H, $C_1$-$C_{12}$-Alkyl, bevorzugt Methyl und Ethyl, Propyl, Butyl sowie Hydroxyalkyl, besonders bevorzugt $-CH_2CH_2OH$, $-CH_2CH_2CH_2OH$ und $-CH_2CH_2CH_2CH_2OH$,

$R^6$ = $C_1$-$C_{12}$-Alkyl, bevorzugt Methyl und Ethyl, Propyl, Butyl, $C_1$-$C_{12}$-geradkettiger und/oder cyclischer mono- oder polyhydroxylierter Kohlenwasserstoffrest, der sich beispielsweise von reduzierten Mono- und Disacchariden ableitet, bevorzugt
$-CH_2CH_2OH$, $-CH_2CH_2CH_2OH$ und $-CH_2CH_2CH_2CH_2OH$, $-CH_2CH(OH)CH_2OH$,

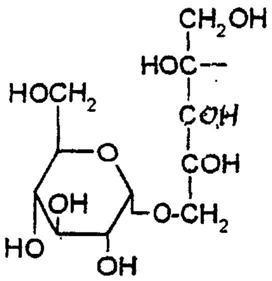
;
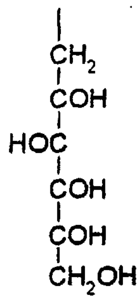

Der Begriff Silikonelastomer umfaßt alle gängigen hydrophoben Silikonelastomere. Bevorzugt ist als Silikonelastomer ein vernetztes Polymer mit einer Viskosität von 2 Pas bis 300 Pas, gemessen bei 20°C, der Formel II

$$M_aSiO_{\frac{4-a}{2}} \qquad (II)$$

mit

M = H, geradkettiges oder verzweigtes oder cyclisches $C_1$-$C_{12}$-Alkyl oder heteroelementsubstituiertes $C_1$-$C_{20}$-Alkyl oder $C_6$-$C_{10}$Aryl und

a= 0, 1, 2 und/oder 3.

Die vernetzten Polysiloxane können als Endgruppen $M_3SiO_{1/2}$-Gruppen und als wiederkehrende Einheiten (i) Gruppen der Formeln $MSiO_{3/2}$ und/oder $SiO_{4/2}$ und gegebenenfalls (ii) $M_2SiO$-Gruppen enthalten. Vernetzt bedeutet dabei, daß mindestens 30 % der angebotenen Vernetzungsstellen verbraucht sind.

Alle zuvor genannten und folgenden Viskositätsangaben wurden bei 20°C ermittelt gemäß DIN 53 015, 53 518 bzw. 53 019 ermittelt. Dabei wurden Viskositäten bis 10 Pas gemäß DIN 53 015, Viskositäten bis 150 Pas nach DIN 53 019 und Viskositäten bis 2000 Pas nach DIN 53 018 ermittelt.

In einer weiteren Ausführungsform der Erfindung weist das Polymer der Formel (II) als Verzweigungsstellen T- und/oder Q-Einheiten auf und ist gegebenenfalls füllstoffhaltig.

In einer bevorzugten Ausführungsform der Erfindung weist das Polymer der Formel (II) $(CH_2)_w$-Brücken, die gegebenenfalls $C_1$-$C_3$-alkylsubstituiert sind, mit w = 2 oder 3 auf.

Nach der gängigen Nomenklatur bedeutet D = $R_2SiO_{2/2}$, T = $RSiO_{3/2}$, M = $R_3SiO_{1/2}$ und Q = $SiO_{4/2}$, (siehe Noll, Chemie und Technologie der Silicone, Verlag Chemie, Weinheim (1968) S. 3).

Ebenfalls bevorzugt ist ein Molverhältnis der Summe der M- und D-Einheiten zu den SiH-Funktionen des Silikonelastomers von 2 bis 500, besonders bevorzugt 2 bis 100, ganz besonders bevorzugt 2 bis 25, ganz, ganz besonders bevorzugt 2 bis 10.

Zudem ist bevorzugt, daß das Verhältnis von SiH :

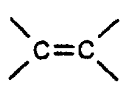

1,1 bis 100:1, besonders bevorzugt 2 bis 50:1, ganz besonders bevorzugt 2 bis 20 : 1 beträgt.

Der Begriff Oberfläche umfaßt dabei Schichtdicken bis zu 50 µm, bevorzugt 10 µm.

In einer weiteren Ausführungsform der Erfindung enthalten die Silikonelastomere Füllstoffe.

Füllstoffe im Sinne der Erfindung sind dabei vorzugsweise gegebenenfalls organisch oberflächenmodifizierte Metalloxide, Zirkoniumdioxid oder Silicat, Titandioxid, Ceroxid, Lithopone, ZnO, Eisenoxid, Diatomeenerde, Calciumcarbonat, pyrogen erzeugtes Siliciumdioxid, mit Silazan behandeltes Siliciumdioxid, gefälltes Siliciumdioxid, Glasfasern, Magnesiumoxid, Chromoxid, Zirkoniumoxid, Aluminiumoxid, α-Quarz, calcinierter Ton, Kohlenstoff, Graphit, synthetische Fasern und ähnliches.

Die bevorzugten Füllstoffe, die in der Masse der vorliegenden Erfindung enthalten sind, sind entweder oberflächenbehandeltes, pyrogenes oder ein gefälltes Siliciumdioxid. Bei einem Verfahren der Oberflächenbehandlung wird z.B. das pyrogene oder gefällte Siliciumdioxid cyclischen Polyorganosiloxanen bei erhöhter Temperatur und erhöhtem Druck ausgesetzt. Ein weiteres Verfahren der Behandlung von Füllstoffen ist eines, bei dem das Siliciumdioxid Siloxanen oder Silanen in Gegenwart einer Aminverbindung ausgesetzt wird.

Ein besonders bevorzugtes Verfahren der Oberflächenbehandlung von Siliciumdioxid-Füllstoffen benutzt Oberflächenbehandlungsmittel aus Methylsilan oder Silazan. Mit Methylsilan oder Silazan oberflächenbehandelte, pyrogene oder gefällte Siliziumdioxid-Füllstoffe haben die Eigenschaft des leichten Fließens, und sie erhöhen auch nicht die geringe Viskosität der ungehärteten Silicon-Zusammensetzung. Nach dem Härten verleihen die Silazan behandelten Siliciumdioxide dem vulkanisierten Elastomer eine verbesserte Reißfestigkeit. Silazan-Behandlungen sind beispielsweise in den US-A 3 635 743 und US-A 3 847 848 offenbart.

Der Füllstoff wird vorzugsweise in einer Konzentration von etwa 5 bis etwa 70 Gewichtsteilen, vorzugsweise 15 bis 50 Gewichtsteilen, bezogen auf 100 Gewichtsteile des eingesetzten Silikonelastomers zugesetzt. Der bevorzugte Füllstoff ist mit Silazan behandeltes, pyrogenes Siliciumdioxid oder Mischungen von Silazan-behandeltem, pyrogenen Siliciumdioxid mit Silazan-behandeltem, gefällten Siliciumdioxid. Diese letztgenannte Mischung ist dann besonders bevorzugt, wenn sie ein Gewichtsverhältnis von pyrogenem Siliciumdioxid zu gefälltem Siliciumdioxid von etwa 25:1 bis etwa 1:1 und vorzugsweise von etwa 10:1 bis etwa 5:1 enthält.

In einer weiteren Ausführungsform der Erfindung weisen die erfindungsgemäßen oberflächlich hydrophilierten Silikonelastomere zwischen der hydrophilen Oberfläche und dem Bulkmaterial eine Oxidschicht auf, wobei dann die Reste $R^1$ vorzugsweise folgende Bedeutung haben:

$R^1$ = $-CH_2OH$,
$-CH_2(OCH_2CH_2)_m(OCH_2CH(CH_3])_n(OCH_2CH[CH_2OH])_oOE$,
$-CH_2OC(O)CH_2CH_2COOH$, $-CH_2OC(O)CH_2CH_2COO^-$,
$-CH_2OC(O)CH=CHCOOH$, $-CH_2OC(O)CH=CHCOO^-$,
$-(CH_2)_pCH(OH)CH_2R^2$, $(CH_2)_pOCH_2CH(OH)CH_2R^2$,
mit

m = 5 bis 100, bevorzugt bis 50, besonders bevorzugt 5 bis 20, ganz besonders bevorzugt 7 bis 20,

n = 1 bis 60, bevorzugt 0 bis 30, besonders bevorzugt 0 bis 10, ganz besonders bevorzugt 1 bis 10,

o = 1 bis 60, bevorzugt 0 bis 50, besonders bevorzugt 5 bis 50, ganz besonders bevorzugt 5 bis 20,

$5 \leq m+n+o \leq 100$, bevorzugt $5 \leq m+n+o \leq 50$,
besonders bevorzugt $7 \leq m+n+o \leq 50$, ganz besonders bevorzugt $7 \leq m+n+o \leq 20$,

p = 1 bis 10, bevorzugt 2 bis 10, besonders bevorzugt 2 bis 4,

q = 0 bis 5, bevorzugt 0 bis 1, besonders bevorzugt 0,

E = $CH_3$, H, $C_4H_9$, $t\text{-}C_4H_9$

$R^2$ = $-NH[(CH_2)_2NH]_sR^4$, $-NH[(CH_2)_3NH]_sR^4$,
$-NHCH_2CH(CH_3)NHR^4$, $N[(CH_2)_2NHR^4]_2$,
$-N[(CH_2)_3NHR^4]_2$,

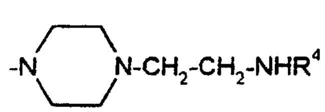
,

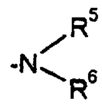

s= 0 bis 10, bevorzugt 1 bis 5, besonders bevorzugt 1 bis 2, ganz besonders bevorzugt 1,

$R^4$ = H, $C_1$-$C_{12}$ geradkettiger und/oder cyclischer mono- oder polyhydroxylierter Kohlenwassersstoffrest, der sich bevorzugt von Hydroxycarbonsäuren, Sacchariden, bevorzugt Mono- und Disacchariden, besonders bevorzugt von oxydierten Mono- und Disacchariden ableitet, ganz besonders bevorzugt oxydierte Mono- und Disaccharide der Formel

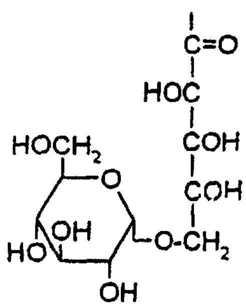

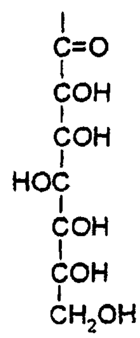

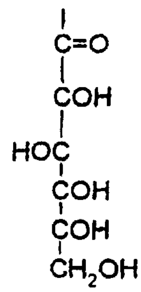

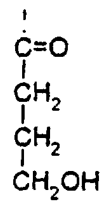

$R^5$ = H, $C_1$-$C_{12}$-Alkyl, bevorzugt Methyl und Ethyl, Propyl, Butyl sowie $C_1$-$C_{12}$-Hydroxyalkyl, besonders bevorzugt $-CH_2CH_2OH$, $-CH_2CH_2CH_2OH$ und $-CH_2CH_2CH_2CH_2OH$,

$R^6$ = $C_1$-$C_{12}$-Alkyl, bevorzugt Methyl und Ethyl, Propyl, Butyl, $C_1$-$C_{12}$ geradkettiger und/oder cyclischer mono- oder polyhydroxylierter Kohlenwasserstoffrest, der sich beispielsweise von reduzierten Mono- und Disacchariden ableitet, bevorzugt $-CH_2CH_2OH$, $-CH_2CH_2CH_2OH$ und $-CH_2CH_2CH_2CH_2OH$, $-CH_2CH(OH)CH_2OH$,

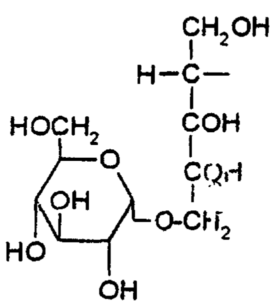

;

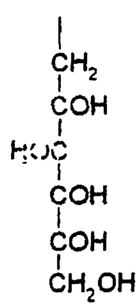

Die Oxidschicht weist dabei vorzugsweise eine Schichtdicke von kleiner 50 µm auf.

In einer weiteren Ausführungsform der Erfindung enthalten die Silikonelastomere insgesamt Füllstoffe. Der Füllstoff befindet sich gegebenenfalls im gesamten Materialbereich.

Gegenstand der Erfindung, ist zudem ein Verfahren zur Herstellung der erfindungsgemäßen oberflächlich hydrophilierten Silikonelastomere, wie es im Anspruch 11 definiert ist.

In einer bevorzugten Ausführungsform der Erfindung erfolgt die Umwandlung der Si-Allkylbindung in $SiO_2$ und/oder SiOH und SiH mittels Plasmabehandlung.

Unter Plasmabehandlung wird die Erzeugung von Elektronen und Ionen im Gemisch durch Einkopplung elektrischer oder magnetischer Felder in einem Sauerstoff enthaltenden oder freisetzendem Reaktandgas in Gegenwart von Silikonelastomeren verstanden.

Dieses Gemisch oder einzelne Bestandteile davon sind dazu geeignet, auf der Oberfläche der Silikonelastomere Bindungen zu spalten und eine Oxidschicht oder SiH-Bindungen aufzubauen. Zum Einsatz kommen bevorzugt HF- oder Mikrowellenplasmageneratoren. Die Zeitspanne der Oxydation im Plasma ist eine Mehrfaktorenoptimierung. Es gehen in bekannter Weise u.a. die Reaktandgaskonzentration, die eingetragene Energie pro Reaktionsraumvolumen und die Frequenz ein. Gewäöhnlich lieft die Einwirkungszeit bevorzugt zwischen wenigen Sekunden bis zu 10 Minuten. Bei der Oxidation des hydrophoben Silikonelastomers im Plasma und anschließender Reaktion mit einem Silan der Formel

$$H_xSiB_{4-x}$$

mit B = Cl, Br, $OCH_3$ und $OC_2H_5$
x = 1-3
werden als ungesättigte Gruppen enthaltenden Polyether, Polyamine und/oder Polyamide vorzugsweise Alkenylamine, ungesättigte Epoxidderivate d.h. z.B. Glycidderivate, wie Allylglycidether, Propinylglycidether, But-(1-en)ylglycidether, But-(1-in)ylglycidether, But-(2-in)yldiglycidether oder Alkenylepoxide, wie z.B. Hexenoxid eingesetzt.
Die Hydrophilierung (Schritt 2) erfolgt vorzugsweise zwischen Raumtemperatur und 150°C. Die Reaktionszeit liegt vorzugsweise zwischen wenigen Minuten und 10 Stunden. Die Reaktionen erfolgen gewöhnlich ohne Lösungsmittel, jedoch können aprotische Lösungsmittel verwendet werden, die das Silikonelastomer nicht oder nur geringfügig anlösen.

In einer weiteren bevorzugten Ausführungsform des erfindungsgemäßen Verfahrens erfolgt anstelle der Umwandlung von Si-Alkyl in SiH direkt der Einsatz eines vernetzten SiH funktionellen Polysiloxans, das hergestellt wurde durch die Umsetzung eines SiH-funktionellen Siloxans mit einem C=C-doppelbindungshaltigen Polysiloxan in Gegenwart eines Übergangsmetallkatalysators.

Das vernetzte SiH-funktionelle Polysiloxan wird im Anschluß an die Umsetzung mit dem olefinisch oder acetylenisch ungesättigten Kohlenwasserstoffatomen vorzugsweise bei einer Temperatur von 60 bis 200°C, besonders bevorzugt 120 bis 160°C getempert.

Als doppelbindungshaltiges Polysiloxan sind dabei alle Polysiloxane einsetzbar, die über mehr als eine CH2=CH-, -CH2CH=CH2 und/oder -CH2CH2CH=CH2 Funktion verfügen. Deren Viskosität, gemessen bei Raumtemperatur (20°C), beträgt vorzugsweise 0,01 bis 200 Pa.s, besonders bevorzugt 0,2 bis 100 Pa.s, bestimmt nach DIN 53 015.

Das doppelbindungshaltige Silan kann durch Hydrolyse geeigneter Halogensilane, bevorzugt Chlorsilane, oder Alkoxysilane, bevorzugt Methoxy- und Ethoxysilane, hergestellt werden. In vorteilhafter Weise erfolgt die Vernetzung durch katalytische Hydrosilylierung ungesättigte Kohlenwasserstoffeinheiten aufweisender Siloxane mit Hydrogensiloxanen. Die benannten Vernetzungsreaktionen sind dem Fachmann aus Silicone, Chemie und Technologie der Silicone, Vulkan-Verlag Chemie Weinheim, Essen (1989), S. 49-63 bekannt.

Als Hydrogensiloxane sind dabei alle gängigen Si-H-funktionellen Siloxane mit einer Viskosität bei 20°C zwischen 0,01 und 5 Pa.s einsetzbar.

Das SiH-funktionelle Siloxan wird dabei vorzugsweise im molaren Überschuß eingesetzt. Das molare Verhältnis von Si-H zu Si-C=C-Doppelbindungs-haltigem Polysiloxan beträgt 2,5 zu 1, bevorzugt 1,5 zu 1, ganz besonders bevorzugt 1,2 zu 1.

Es ist erfindungswesentlich, daß in diesem Siloxan zunächst SiH-Funktionen erhalten bleiben, deren oberflächennaher Anteil in nachfolgenden Hydrosilylierungen für die Funktionalisierungen genutzt werden kann. Dies wird vorzugsweise durch einen molaren Überschuß von SiH : C=C von 1,1 bis 100:1, besonders bevorzugt 2 bis 50:1, ganz besonders bevorzugt 2 bis 20:1, erreicht.

Als Übergangsmetall-Katalysator werden vorzugsweise Pt-Katalysatoren nach Speier, Karstedt und Lamoreaux (B. Marcinec, Comprehensive Handbook on Hydrosilylation, first edition, Pergamon Press 1992, S. 41-49) eingesetzt. Beispielsweise wird Platin in Mengen von $10^{-3}$ bis $10^{-6}$ mol pro mol ungesättigter Funktion zugesetzt und die Vernetzungsreaktion zwischen Raumtemperatur (20°C) und 100°C ausgeführt.

Die Oberflächenmodifizierung der im ersten Schritt gebildeten SiH-haltigen Siloxanverbindung nach beiden beschriebenen Verfahren erfolgt in Abhängigkeit von der gewünschten Funktionalisierung ein- oder mehrstufig.

Einstufige Modifizierungen sind bevorzugt, wenn entsprechend fünktionalisierte olefinisch oder acetylenisch ungesättigte Derivate zur Verfügung stehen.

Dies trifft in vorteilhafter Weise auf Verbindungen der Typen

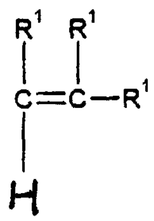 oder 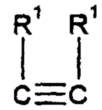

zu, wobei $R^1$ die oben angegebene Bedeutung hat.

Beispiele für heteroelementsubstituierte ungesättigte Kohlenwasserstoffe sind Alkenole und Alkinole, Alkendiole und Alkindiole [z.B. Allylalkohol, Propinol, But-(1-en)ol, But-(2-en)ol, Hex-(1-en)ol, Hex-(1-in)ol, But-(2-en)diol, But-(2-in)diol] und deren Oligooxyalkylenderivate, bevorzugt Ethoxylierungsund/oder Propoxylierungsprodukte. Für diese Oligooxyalkylenderivate kann es von Vorteil sein, die endständige Hydroxylgruppe zu blockieren, z.B. als Methoxy-, Ethoxy- und Butoxyendgruppe. Weiterhin kommen bevorzugt Alkenylamine (z.B. Allylamin oder die Umsetzungsprodukte von ungesättigten Epoxiden mit primären und sekundären Aminen) und teilfluorierte Alkene (z.B. des Typs $-CH_2=CH(CF_2CF_2)_{0-17}CF_3$) und teilfluorisrte ungesättigte Ether, wie z.B. $-CH_2=CHCH_2OCH_2(CF_2)_{1-20}CF_2H$ und $-CH_2=CHCH_2OCH_2CH_2(CF_2)_{0-17}CF_3$, in Betracht.

Diese Verbindungen werden in Masse oder in polar aprotischen Lösungsmitteln mit der zu funktionalisierenden SiH-haltigen Oberfläche in Gegenwart eines Hydrosilylierungskatalysators umgesetzt. Die Reaktion findet vorzugsweise im Temperaturbereich von 20°C bis 150°C statt.

Die Reaktionszeiten sind edukt- und temperaturabhängig und liegen im Bereich von 1 min bis zu 10 Stunden.

Alternativ dazu kann eine mehrstufige Modifizierung der Oberfläche vorgenommen werden.

In der ersten Stufe erfolgt die Einführung reaktiver Strukturen, wobei als Reaktionspartner bevorzugt die vorstehend benannten Alkenylamine, Alkenyldicarbonsäureanhydride (z.B. 5-Norbornen-2,3-dicarbonsäureanhydrid) und ungesättigte Epoxidderivate [z.B. Glycidderivate, wie Allylglycidether, Propinylglycidether, But-(1-en)ylglycidether, But-(1-in)ylglycidether, But-(2-in)yldiglycidether oder Alkenylepoxide, wie z.B. Hexenoxid herangezogen werden.

In der zweiten Stufe werden diese reaktiven Strukturen mit geeigneten nucleophilen oder elektrophilen Agenzien zu den Zielstrukturen umgesetzt.

Als geeignet für diese Zwecke erweisen sich beispielsweise die nucleophilen Amine und Aminoamide der Strukturen

$$HN[(CH_2)_2NHR^4]_2HN[(CH_2)_3NHR^4]_2,$$

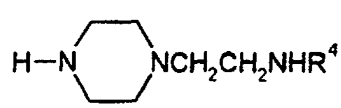 , 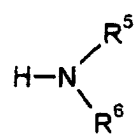 ,

wobei $R^4$, $R^5$ $R^6$ die bereits angegebene Bedeutung besitzen.

Neben den Aminoalkoholen sind die von reduzierenden Kohlehydraten abgeleiteten polyhydroxylierten Amine, wie z.B. Glucamin, Maltamin, Isomaltamin, N-Alkylglucamine, wie N-Methylglucamin, N-Hydroxyalkylglucamine, wie N-(Hydroxyethyl)glucamin und aus primär-sekundären Polyaminen und Carbonsäurelactonen synthetisierbaren hydroxylierten Aminoamide, gemäß Appl. Organomet. Chem., 10(1996), 421 und 437, von besonderem Interesse.

Bevorzugte elektrophile Reaktionspartner sind z.B. aktivierte Carbonsäurederivate, speziell Lactone. Es kann hierbei auf nicht- (z.B. γ-Butyrolacton), mono- (z.B. α-Hydroxy-γ-butyrolacton) und/oder polyhydroxylierte Lactone zurückgegriffen werden. Für den Fall einer möglichst starken Hydrophilierung greift man vorzugsweise auf polyhydroxylierte Oxydationsprodukte von Mono- und Disacchariden (z.B. Gluconsäurelacton, Glucopyranosylarabonsäurelacton) zurück.

Gegenstand der vorliegenden Erfindung sind zudem oberflächlich hydrophilierte Silikonelastomere, erhältlich nach den erfindungsgemäßen Verfahren.

Ein weiterer Gegenstand der Erfindung ist die Verwendung der oberflächlich hydrophilierten Silikonelastomere zur Herstellung von Formteilen für den medizinischen und medizintechnischen Bereich, z.B. als Katheter, Wundabdeckungsmaterialien und Injektionsports.

Die nachfolgenden Beispiele beschreiben die erfindungsgemäße Oberflächenhydrophilierung von Silikonelastomeren durch kovalente Verknüpfung mit Ethylenoxidderivaten, ohne dabei auf diese beschränkt zu sein.

## Ausführungsbeispiele

### Beispiel 1

0,8 g (5,32 · $10^{-5}$ mol, 10,6 · $10^{-5}$ mol C=C) eines α,ω-vinylterminierten Polydimethylsiloxanes der durchschnittlichen Zusammensetzung

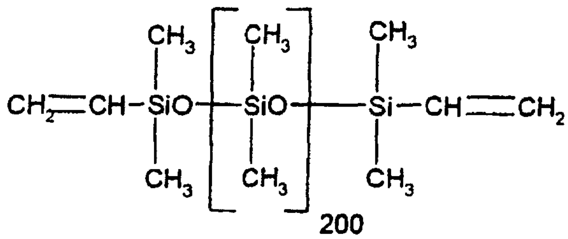

$M^{Vi}D_{200}M^{Vi}$

und 0,1 g (3,68 · $10^{-5}$ mol, 1,13 · $10^{-3}$ mol SiH) eines Hydrogenpolysiloxanes der durchschnittlichen statistischen Zusammensetzung

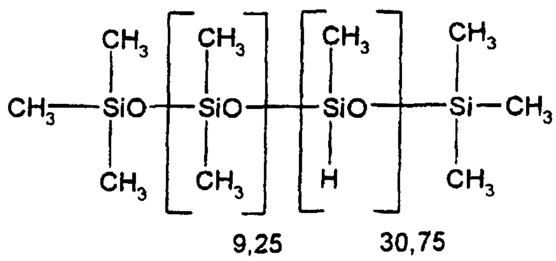

$M_2D_{9,25}D^H_{30,75}$

wurden unter Argon in einem zweiteiligen Glasreaktor mit ebener Bodenplatte miteinander vereinigt. 10 mg einer 3,5 % Platin enthaltenden Katalysatorlösung nach Lamoreaux (US-A 3 220 972) wurden eingemischt und die Masse 30 Minuten bei Raumtemperatur und 1 Stunde bei 50°C vernetzt. Aufgesetzte Wassertropfen bildeten einen goniometrisch bestimmten Randwinkel von 94° (Durchschnitt von 8 Messungen).

Im Glasreaktor wurde unter Argon auf die Oberfläche des Polysiloxannetzwerkes eine Mischung aus 10 mg Lamoreaux-Katalysator und 0,8 g eines allylmodifizierten Ethylenoxidderivates der durchschnittlichen Zusammensetzung $CH_2=CHCH_2O(CH_2CH_2O)_{7,5}OCH_3$ aufgebracht. Anschließend erfolgte für 6 Stunden Erwärmung auf 100°C. Nach Beendigung der Reaktion wurde das überschüssige Ethylenoxidderivat mit Methanol, Wasser und wieder Methanol abgewaschen und das oberflächenmodifizierte Siloxannetzwerk anschließend bei Raumtemperatur getrocknet. Unmittelbar nach Beendigung der Trocknung aufgesetzte Wassertropfen lieferten einen Randwinkel von 69°. Zum Nachweis der Langzeitstabilität wurde die Randwinkelmessung nach 7 bzw. 42 Tagen wiederholt. Nach sieben Tagen wurde ein Randwinkel von 58° und nach 42 Tagen von 40° gefunden. Dies belegt die Dauerhaftigkeit der Hydrophilie.

**Beisoiele 2 bis 7**

Analog zu Beispiel 1 wurden die in der Tabelle 1 angegebenen H-Siloxane mit den dort angegebenen Vinylsiloxanen umgesetzt.

Einsatzmengen sowie Ergebnisse sind in Tabelle 1 aufgelistet.

**Tabelle 1**

| Beispiel | H-Siloxan | Vinylsiloxan | Vi:SiH:ΣD+M | $CH_2$=$CHCH_2(OCH_2CH_2)_nOCH_3$ | Randw. nach Vernetzung [°] | Randw. nach EO-Add. [°] | Randwinkel Langzeit [°] |
|---|---|---|---|---|---|---|---|
| 1 | $M_2D_{9,25}D^H{}_{30,75}$ | $M^{Vi}D_{200}M^{Vi}$ | 1 : 10,5 : 103 | 7,5 | 94 | 69 | 58(7); 40(42) |
| 2 | $M_2D_{9,25}D^H{}_{30,75}$ | $M^{Vi}D_{200}M^{Vi}$ | 1 : 10,5 : 103 | 32 | | 45 | 49(7) |
| 3 | $M_2D_{20}D^H{}_{10}$ | $M^{Vi}D_{19,3}M^{Vi}$ | 1 : 7 : 23,5 | 7,5 | 90 | | |
| 4 | $M_2D_{20}D^H{}_5$ | $M^{Vi}D_{19,3}M^{Vi}$ | 1 : 8 : 40 | 7,5 | | <10 | <10(30) |
| 5 | $M_2D_{20}D^H{}_5$ | $M^{Vi}D_{19,3}M^{Vi}$ | 1 : 5 : 30 | 7,5 | | <10 | |
| 6 | $M_2D_{20}D^H{}_5$ | $M^{Vi}D_{19,3}M^{Vi}$ | 1 : 2 : 18 | 7,5 | | 20 | 21(14) |
| 7 | $M^HD^H{}_7D_{200}M^H$ | $M^{Vi}D_{19,3}M^{Vi}$ | 1 : 9 : 223 | 7,5 | | 48 | 45(7) |

Add. = Addition

* statistische Verteilung (EO-Einheiten pro Allylgruppierung)

**Beispiel 8**

Streifen aus vernetztem Polydimethylsiloxan auf Basis von vinylterminierten Polysiloxanen der Viskosität 10 bzw. 65 Pa.s, gemessen bei 20°C nach DIN 53 015, mit 23 Gew.-%, bezogen auf die Menge des eingesetzten Polymers, eingemischter trimethylsilanolhydrophilierten pyrogenen Kieselsäure (Viskosität: 600 Pa.s, gemessen bei 20°C nach DIN 53 018, bei z = 10 sec-1), deren Vernetzung des Kautschuks mit Hilfe eines 7,3 mmol/g Si-H-Gruppen enthaltenden Polysiloxanpolymers der Viskosität 40 mPa.s erfolgte, mit einer Dicke von 2 mm wurden mehrmals in Diethylether vorgereinigt. Anschließend wurden diese Streifen für 20 Minuten in einem Plasmacleaner PCD-32G (Harrick, 100 Watt Leistung) mit einem H2-Plasma oberflächlich hydriert. Nach Entnahme aus dem Plasmacleaner (Harrick, 100 Watt Leistung) wurden die oberflächlich SiH-funktionalisierten Streifen zur Ausführung der Oberflächenhydrosilylierung in eine Mischung aus 7 g
$CH2=CHCH2(OCH2CH2)_nOCH3$ (Uniox PKA-5007), Molgewicht 400 g/mol, NOF Corporation) und 30 mg eines 3 % Platin enthaltenden Lamoreaux-Katalysators (USA 32 20 972) eingebracht. Die Reaktion erfolgte unter Schutzgas für 7 Stunden bei 80°C. Nach Beendigung der Reaktion wurden die Streifen in Ethanol und Wasser intensiv gespült und an der Luft getrocknet.
Die erhaltenen Strukturen wurden mit FITIR-ATR Spektroskopie gesichert.
Spektrum 1 zeigt das vernetzte PDMS im unmodifizierten Zustand.
Spektrum 2 zeigt das Material nach SiH-Funktionalisierung im H2-Plasma. Deutlich ist die SiH-Bande bei 2236 $cm^{-1}$ zu erkennen.
Spektrum 3 zeigt das Material nach der Hydrosilylierung mit dem allylmodifizierten Ethylenoxidderivat. Das starke Signal bei 2878 $cm^{-1}$ geht auf die -CH2-Gruppen zurück. Ein schwächeres Signal bei 2236 $cm^{-1}$ deutet an, daß nicht alle SiH-Funktionen umgesetzt wurden.

Randwinkel gegen Wasser:

| | |
|---|---|
| unmodifiziertes PDMS | 97° (Vorrückrandwinkel |
| nach Hydrosilylierung mit dem Ethylenoxidderivat | 42° (Vorrückrandwinkel) |

Beispiel 9

In einem Kneter wurden 700 Teile vinylendgestopptes Polydimethylsiloxan mit einer Viskosität von 1 000 mPa.s bei 20°C mit 40 Teilen Hexamethyldisilazan und 35 Teilen Wasser gemischt und anschließend mit 225 Teilen einer pyrogen hergestellten Kieselsäure mit einer spezifischen Oberfläche von 300 m'-/g nach BET zu einer homogenen Masse verknetet. Die Mischung wurde zunächst auf 130°C erwärmt und 1,5 Stunden im geschlossenen Kneter gerührt und danach bei 160°C unter Vakuum 1 Stunde von Wasser und überschüssigem Hexamethyldisilazan befreit. Nach dem Erkalten erhält man einen Vaseline-ähnlichen Compound. Basierend auf einer solchen Zubereitung wurden Streifen von 2 mm Dicke vulkanisiert (10'/170°C). Diese Streifen wurden mehrmals mit Diethylether vorgereinigt. Anschließend wurden diese Streifen für 3 Minuten in einem Plasmacleaner PDC-32G (Harrick, 100 Watt Leistung) mit einem Luftplasma oberflächlich oxydiert. Nach Entnahme aus dem Plasmacleaner erfolgte in einem Glasreaktor die Adsorption von Trichlorsilan auf die aktivierte Oberfläche. Hierzu wurde der Reaktionsraum mit den darin befindlichen Streifen zunächst evakuiert (50 mm Hg) und bei Raumtemperatur aus einem Vorratsgefäß Trichlorsilan bis zur Einstellung des Gleichgewichtsdruckes eingeströmt. Drei derartige Zyklen wurden in einem Gesamtzeitraum von einer Stunde realisiert. Nach abschließender Entfernung aller flüchtigen Komponenten im Vakuum wurden die mit einem SiH funktionalisierten Polysiloxannetzwerk überzogenen Streifen zur Ausführung einer Oberflächenhydrosilylierung in eine Mischung aus 7 g $CH_2=CHCH_2(OCH_2CH_2)_nOCH3$ (Uniox PKA-5007, Molgewicht 500 g/mol, NOF Corporation) und 30 mg eines 3 % Platin enthaltenden Lamoreaux Katalysators (US3 220 972) eingebracht. Die Reaktion erfolgte unter Schutzgas für vier Stunden bei 80°C. Nach Beendigung der Reaktion wurden die Streifen mit Ethanol und Wasser intensiv gespült und an Luft getrocknet.
Die Strukturen wurden mit FTIR-ATR Spektroskopie gesichert.
Spektrum I zeigt das vernetzte Polysiloxan im unmodifizierten Ausgangszustand.

Spektrum 2 zeigt das Material nach Adsorption und Vernetzung von $Cl_3SiH$. Deutlich ist die Si-H-Bande bei 2 252 $cm^{-1}$ zu erkennen.

Spektrum 3 zeigt das Material nach der Hydrosilylierung mit dem allylmodifizierten Ethylenoxidderivat. Starke Signale bei 2 878 und 2 827 $cm^{-1}$ gehen auf die $-CH_2$-Einheiten der Ethylenoxidkette zurück. Das noch vorhandene

Signal bei 2 252 $cm^{-1}$ zeigt, daß nicht alle SiH-Funktionen im Polysiloxannetzwerk reagiert haben.

Randwinkel gegen Wasser

| | |
|---|---|
| unmodifiziertes PDMS LSR 2040 | 97° (Vorrückrandwinkel) |
| nach Adsorption und Vernetzung von $Cl_3SiH$ | 77° (Vorrückrandwinkel) |
| nach Hydrosilylierung mit Ethylenoxidderivat | 54° (Vorrückrandwinkel), der Rückzugsrandwinkel von >20° zeigt an, daß eine starke Hysterese auftritt |

**Patentansprüche**

1. Selektiv oberflächlich hydrophilierte Silikonelastomere, deren in der Oberfläche sitzende Siliziumatome zu I bis 100 % in Form der Einheiten der Formel

$$[Z\ X\ Y\ SiO_{1/2}] \qquad (I)$$

vorliegen, in denen

X = ein geradkettiges oder verzweigtes oder cyclisches $C_1$-$C_{12}$-Alkyl oder heteroelementsubstituiertes $C_1$-$C_{20}$-Alkyl oder $C_6$-$C_{10}$-Aryl,

Z = $O_{1/2}$ oder X und

Y =

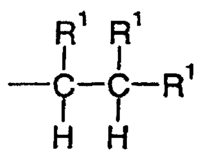

oder

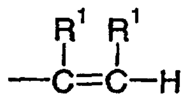

mit

$R^1$ = H, geradkettiges oder verzweigtes oder cyclisches $C_1$-$C_{12}$-Alkyl oder heteroelementsubstituiertes $C_1$-$C_{20}$-Alkyl oder $C_6$-$C_{10}$-Aryl,
wobei die Reste $R^1$ innerhalb eines Moleküls gleich oder ungleich sein können,
mit der Maßgabe, dass mindestens einer der Reste $R^1$ ausgewählt ist aus:

$R^1$ = $-CH_2OH$,
$-CH_2(OCH_2CH_2)_m(OCH_2CH[CH_3])_n(OCH_2CH[CH_2OH])_oOE$,
$-CH_2OC(O)CH_2CH_2COOH$,
$-CH_2OC(O)CH_2CH_2COO^-$,
$-CH_2OC(O)CH{=}CHCOOH$,
$-CH_2OC(O)CH{=}CHCOO^-$,
$-(CH_2)_pCH(OH)CH_2R^2$,
$-(CH_2)_pOCH_2CH(OH)CH_2R^2$,
$-(CH_2)_qR^3$,
$-(CH_2)_pO(CH_2)_pR^3$,

mit

m = 0 bis 100,
n = 0 bis 60,
o = 0 bis 60, mit

$0 \le m+n+o \le 100$,

p = 1 bis 10,
q = 0 bis 5,
E = $CH_3$, H, $C_4H_9$, t-$C_4H_9$

$R^2$ = $-NH[(CH_2)_2NH]_sR^4$,
$-NH[(CH_2)_3]_sR^4$,
$-NHCH_2CH(CH_3)NHR^4$,
$-N[(CH_2)_2NHR^4]_2$,
$-N[(CH_2)_3NHR^4]_2$,

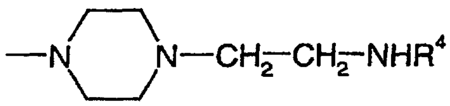

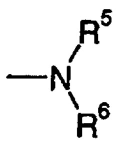

$R^3$= $-(CF_2)_tCF_3$,
$-(CF_2)_tCF_2H$

mit
s = 0 bis 10,
t = 0 bis 19,

$R^4$ = H, $C_1$-$C_{12}$ geradkettiger und/oder cyclischer mono- oder polyhydroxylierter Kohlenwasserstoffrest,

$R^5$ = H, $C_1$-$C_{12}$-Alkyl, Hydroxyalkyl und

$R^6$ = $C_1$-$C_{12}$-Alkyl, $C_1$-$C_{12}$ geradkettiger und/oder cyclischer mono- oder polyhydroxylierter Kohlenwasserstoffrest der Formel
$-CH_2CH_2OH$,
$-CH_2CH_2CH_2OH$ und
$-CH_2CH_2CH_2CH_2OH$,
$-CH_2CH(OH)CH_2OH$,

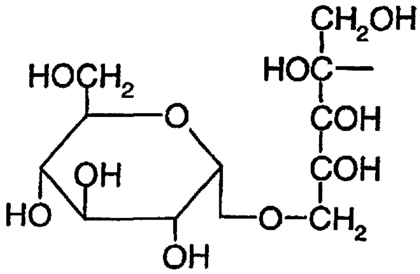
,
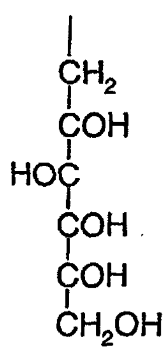
.

**2.** Selektiv oberflächlich hydrophilierte Silikonelastomere nach Anspruch 1, **dadurch gekennzeichnet, dass** der Rest $R^4$ oxydierte Mono- und Disaccharide der Formel

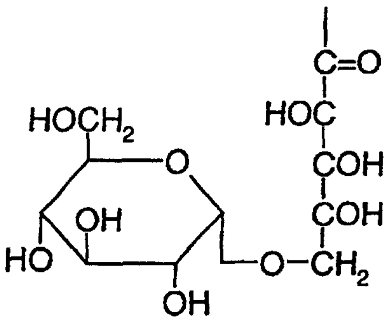 , 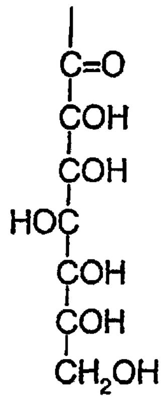 , 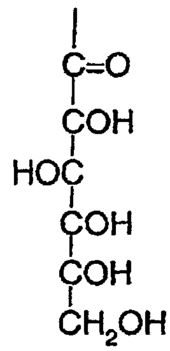 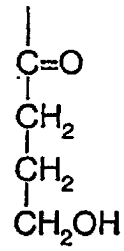

darstellt.

**3.** Selektiv oberflächlich hydrophilierte Siliconelastomere nach Anspruch 1, **dadurch gekennzeichnet, dass** das Silikonelastomer ein vernetztes Polymer mit einer Viskosität von 2 Pa·s bis 300 Pa·s, ermittelt bei 20°C gemäß DIN 53 015, 53 518 bzw. 53 019, ist und aus Einheiten der Formel II

$$M_aSiO_{\frac{4-a}{a}} \quad \text{(II)}$$

mit

M = H, geradkettiges oder verzweigtes oder cyclisches $C_1$-$C_{12}$-Alkyl, heteroelementsubstituiertes $C_1$-$C_{20}$-Alkyl oder $C_6$-$C_{10}$-Aryl und
a = 0, 1, 2 und/oder 3 ist.

**4.** Selektiv oberflächlich hydrophilierte Silikonelastomere nach Anspruch 3, **dadurch gekennzeichnet, dass** das Polymer (II) als Verzweigungsstelle T- und/oder Q-Einheiten aufweist und gegebenenfalls füllstoffhaltig ist.

**5.** Selektiv oberflächlich hydrophilierte Silikonelastomere nach Anspruch 3 oder 4, **dadurch gekennzeichnet, dass** das Polymer der Formel (II) $C_1$-$C_3$-alkylsubstituierte $(CH_2)_w$-Brücken mit w=2 oder 3 zwischen jeweils zwei Siliciumatomen aufweist, die aus der Reaktion von SiH-Strukturen mit Vinyl- der Allylverbindungen resultieren.

**6.** Selektiv oberflächlich hydrophilierte Silikonelastomere nach einem oder mehreren der Ansprüche 1 bis 5, **dadurch gekennzeichnet, dass** X=1,1,1-Trifluorpropyl ist.

**7.** Selektiv oberflächlich hydrophilierte Silikonelastomere nach einem oder mehreren der Ansprüche 1, 3, 4, 5 oder 6, **dadurch gekennzeichnet, dass** diese zwischen der hydrophilierten Oberfläche und dem Bulkmaterial eine Oxidschicht aufweisen, wobei der Rest $R^1$ wie im Anspruch 1 definiert ist.

**8.** Selektiv oberflächlich hydrophilierte Silikonelastomere nach Anspruch 7, **dadurch gekennzeichnet dass** der Rest $R^4$ Mono- und Disaccharide gemäß Anspruch 2 darstellt.

**9.** Selektiv oberflächlich hydrophilierte Silikonelastomere nach Anspruch 7, **dadurch gekennzeichnet, dass** der Rest $R^5$ $-CH_2CH_2OH$, $-CH_2CH_2CH_2OH$ und $-CH_2CH_2CH_2CH_2OH$ ist.

**10.** Selektiv oberflächlich hydrophilierte Silikonelastomere nach Anspruch 7, **dadurch gekennzeichnet, dass** der Rest $R^6$ $-CH_2CH_2OH$, $-CH_2CH_2CH_2OH$ und $-CH_2CH_2CH_2CH_2OH$, sowie $-CH_2CH(OH)CH_2OH$,

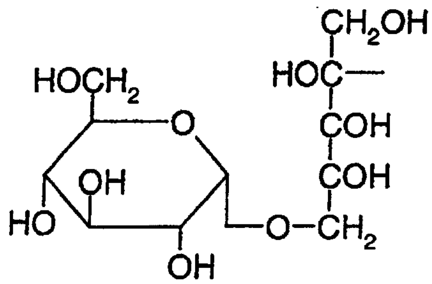

,

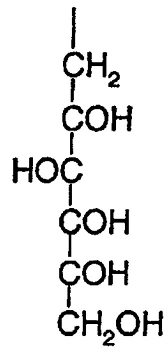

ist.

**11.** Verfahren zur Herstellung von selektiv oberflächlich hydrophilierten Silikonelastomeren nach Anspruch 1, **dadurch gekennzeichnet, dass** in einem hydrophoben Silikonelastomer

1. mindestens eine Si-Alkylbindung an der Oberfläche in a) SiOH und/oder $SiO_2$ oder b) in SiH umgewandelt wird,
und dann

2. a) SiOH und/oder $SiO_2$ an der Oberfläche entweder mit einem Silan der Formel

$$H_xSiB_{4-x}$$

mit B = Cl, Br, $OCH_3$ und $OC_2H_5$ und
x = 1-3
und dann im Anschluß daran
dieses Umsetzungsprodukt mit funktionellen Olefinen oder Acetylenen in Gegenwart eines Platinkatalysators umgesetzt wird, oder SiOH oder $SiO_2$, an der Oberfläche mit einem Silan der Formel

$$Y_xSiB_{4-x}$$

mit B = Cl, Br, $OCH_3$ und $OC_2H_5$ und
x = 1-3
und worin Y wie im Anspruch 1 definiert ist.
umgesetzt wird,
sowie

2. b) SiH an der Oberfläche mit olefinisch oder acetylenisch ungesättigten Kohlenwasserstoffen mit der allgemeinen Struktur

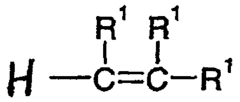

oder

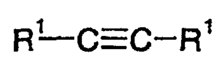

wobei die Reste $R^1$ entsprechend der Definition von $R^1$ im Anspruch 1 definiert sind.
sowie

3. anstelle der Umwandlung von Si-Alkyl in SiH direkt ein vernetztes SiHfunktionelles Polysiloxan eingesetzt

wird, das durch die Umsetzung eines SiH-funktionellen Siloxans mit einem C=C-doppelbindungshaltigen Polysiloxan in Gegenwart eines Übergangsmetallkatalysators hergestellt wurde.

**12.** Verfahren zur Herstellung von selektiv oberflächlich hydrophilierten Silikonelastomeren nach Anspruch 11, **dadurch gekennzeichnet, dass** die funktionellen Olefine oder Acetylene, mit denen das Produkt der Umsetzung von SiOH und/oder $SiO_2$ mit dem Silan der Formel $H_xSiB_{4-x}$ umgesetzt wird, ungesättigte Gruppen enthaltende Polyether, Polyamine und/oder Polyamide sind, wobei x und B wie im Anspruch 11 definiert sind.

**13.** Verfahren nach Anspruch 11, **dadurch gekennzeichnet, dass** die Umwandlung der Si-Alkylbindung in $SiO_2$ und/oder SiOH und SiH mittels Plasmabehandlung erfolgt.

**14.** Verfahren nach Anspruch 11, **dadurch gekennzeichnet, dass** das vernetzte SiH-funktionelle Polysiloxan im Anschluß an die Umsetzung mit dem olefinisch oder acetylenisch ungesättigten Kohlenwasserstoffen bei einer Temperatur von 60 bis 200°C getempert wird.

**15.** Verwendung der selektiv oberflächlich hydrophilierten Silikonelastomere nach einem oder mehreren der Ansprüche 1 bis 10 zur Herstellung von Artikeln für den medizinischen und medizinisch-technischen Bereich.

**16.** Artikel für den medizinischen und medizinisch-technischen Bereich, die unter Verwendung der selektiv oberflächlich hydrophilierten Silikonelastomere nach einem oder mehreren der Ansprüche 1 bis 10 hergestellt wurden.

## Claims

**1.** Selectively surface-hydrophilized silicone elastomers, whose silicon atoms sitting in the surface are to the extent of from 1 to 100% in the form of units of the formula

$$[Z\ X\ Y\ SiO_{1/2}] \qquad (I)$$

in which

X = straight-chain or branched or cyclic $C_1$-$C_{12}$-alkyl or hetero-element-substituted $C_1$-$C_{20}$-alkyl or $C_6$-$C_{10}$-aryl,

Z = $O_{1/2}$ or X, and

Y =

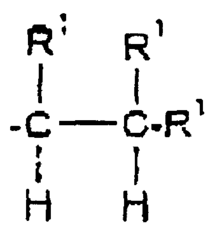

or

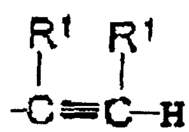

where

$R^1$ =H, straight-chain or branched or cyclic $C_1$-$C_{12}$-alkyl or hetero-element-substituted $C_1$-$C_{20}$-alkyl or $C_6$-$C_{10}$-aryl,
where the radicals $R^1$ within a molecule may be identical or different,
with the proviso that at least one of the radicals $R^1$ is selected from:

$R^1$ = $-CH_2OH$,
$-CH_2(OCH_2CH_2)_m(OCH_2CH[CH_3])_n(OCH_2CH[CH_2OH])_oOE$,
$-CH_2OC(O)CH_2CH_2COOH$,
$-CH_2OC(O)CH_2CH_2COO^-$,

$-CH_2OC(O)CH=CHCOOH$,
$-CH_2OC(O)CH=CHCOO^-$,
$-(CH_2)_pCH(OH)CH_2R^2$,
$-(CH_2)_pOCH_2CH(OH)CH_2R^2$,
$-(CH_2)_qR^3$,
$-(CH_2)_pO(CH_2)_pR^3$,

where

m = from 0 to 100,

n = from 0 to 60,

o = from 0 to 60, where
$0 \le m + n + o \le 100$,

p = from 1 to 10,

q = from 0 to 5,

E = $CH_3$, H, $C_4H_9$ or t-$C_4H_9$,

$R^2$ = $-NH[(CH_2)_2NH]_sR^4$,
$-NH[(CH_2)_3NH]_sR^4$,
$-NHCH_2CH(CH_3)NHR^4$,
$N[(CH_2)_2NHR^4]_2$,
$-N[(CH_2)_3NHR^4]_2$,

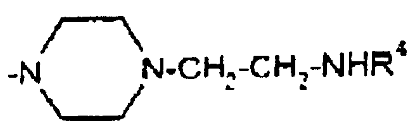

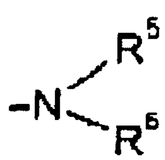

$R^3$ = $-(CF_2)_tCF_3$,
$-(CF_2)_tCF_2H$

where

s = from 0 to 10,

t = from 0 to 19,

$R^4$ = H, a straight-chain and/or cyclic mono- or polyhydroxylated $C_1$-$C_{12}$-hydrocarbon radical,

$R^5$ = H, $C_1$-$C_{12}$-alkyl, hydroxyalkyl and

$R^6$ = $C_1$-$C_{12}$-alkyl, a straight-chain and/or cyclic mono- or polyhydroxylated $C_1$-$C_{12}$-hydrocarbon radical of the formula
$-CH_2CH_2OH$,
$-CH_2CH_2CH_2OH$ and

$-CH_2CH_2CH_2CH_2OH$,
$-CH_2CH(OH)CH_2OH$,

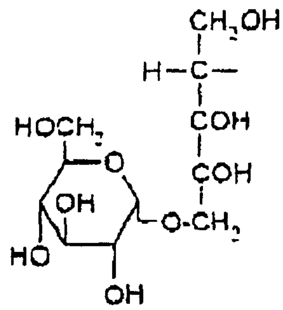 ; 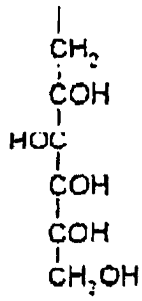 .

2. Selectively surface-hydrophilized silicone elastomers according to Claim 1, **characterized in that** the radical $R^4$ constitutes oxidized mono- and disaccharides of the formula

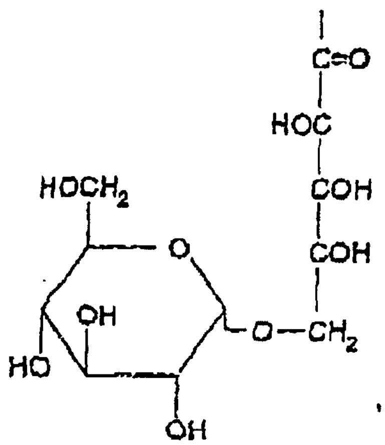 , 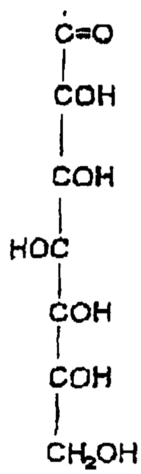 , 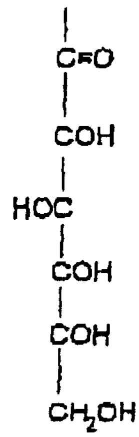 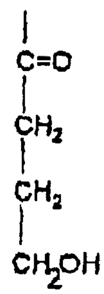

3. Selectively surface-hydrophilized silicone elastomers according to Claim 1, **characterized in that** the silicone elastomer is a crosslinked polymer having a viscosity of from 2 Pa·s to 300 Pa·s, determined at 20°C in accordance with DIN 53 015, 53 518 or 53 019, and comprises units of the formula II

$$M_aSiO_{\frac{4-a}{2}} \qquad (II)$$

where

M = H, straight-chain or branched or cyclic $C_1$-$C_{12}$-or $C_6$-$C_{10}$-aryl and

a = 0, 1, 2 and/or 3.

4. Selectively surface-hydrophilized silicone elastomers according to Claim 3, **characterized in that** the polymer (II) has T and/or Q units as branching points and optionally contains fillers.

5. Selectively surface-hydrophilized silicone elastomers according to Claim 3 or 4, **characterized in that** the polymer of the formula (II) has $C_1$-$C_3$-alkyl-substituted $(CH_2)_w$ bridges, where w = 2 or 3, in each case between two silicon atoms which result from the reaction of SiH structures with vinyl the allyl compounds.

6. Selectively surface-hydrophilized silicone elastomers according to one or more of Claims 1 to 5, **characterized in that** X = 1,1,1-trifluoropropyl.

7. Selectively surface-hydrophilized silicone elastomers according to one or more of Claims 1, 3, 4, 5 or 6, **characterized in that** these have an oxide layer between the hydrophilized surface and the bulk material, where the radical $R^1$ is as defined in Claim 1.

8. Selectively surface-hydrophilized silicone elastomers according to Claim 7, **characterized in that** the radical $R^4$ constitutes mono- or disaccharides according to Claim 2.

9. Selectively surface-hydrophilized silicone elastomers according to Claim 7, **characterized in that** the radical $R^5$ is $-CH_2CH_2OH$, $-CH_2CH_2CH_2OH$ and $-CH_2CH_2CH_2CH_2OH$.

10. Selectively surface-hydrophilized silicone elastomers according to Claim 7, **characterized in that** the radical $R^6$ is $-CH_2CH_2OH$, $-CH_2CH_2CH_2OH$, and $-CH_2CH_2CH_2CH_2OH$ as well as $-CH_2CH(OH)CH_2OH$,

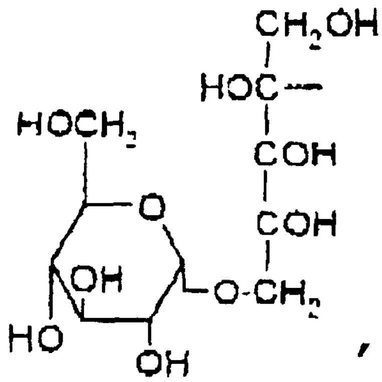
,
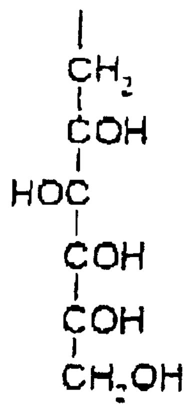
.

11. Process for the preparation of the selectively surface-hydrophilized silicone elastomers according to Claim 1, **characterized in that**, in a hydrophobic silicone elastomer,

1. at least one Si-alkyl bond at the surface is converted into a) SiOH and/or $SiO_2$ or b) into SiH and then

2. a) SiOH and/or $SiO_2$ at the surface is reacted either with a silane of the formula

$$H_xSiB_{4-x}$$

where B = Cl, Br, $OCH_3$ or $OC_2H_5$ and
x = 1-3
and then subsequently thereto
this reaction product is reacted with functional olefins or acetylenes in the presence of a platinum catalyst, or SiOH or $SiO_2$ at the surface is reacted with a silane of the formula

$$Y_xSiB_{4-x}$$

where B = Cl, Br, $OCH_3$ or $OC_2H_5$ and
x = 1-3
and where Y is as defined in Claim 1,
and

2b) SiH at the surface is reacted further with olefinically or acetylenically unsaturated hydrocarbons having

the general structure

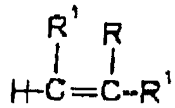

or

$$R^1\text{-}C\equiv C\text{-}R^1$$

where the radicals $R^1$ are defined in accordance with the definition of $R^1$ in Claim 1,

3. instead of the converion of Si-alkyl into SiH directly, a crosslinked SiH-functional polysiloxane is employed which has been prepared by the reaction of an SiH-functional siloxane with a polysiloxane containing a C=C double bond in the presence of a transition-metal catalyst.

**12.** Process for the preparation of selectively surface-hydrophilized silicone elastomers according to Claim 11, **characterized in that** the functional olefins or acetylenes with which the product of the reaction of SiOH and/or $SiO_2$ with the silane of the formula $H_xSiB_{4-x}$ is reacted are polyethers, polyamines and/or polyamides containing unsaturated groups, where x and B are as defined in Claim 11.

**13.** Process according to Claim 11, **characterized in that** the conversion of the Si-alkyl bond into $SiO_2$ and/or SiOH and SiH is carried out by means of plasma treatment.

**14.** Process according to Claim 11, **characterized in that** the crosslinked SiH-functional polysiloxane is conditioned at a temperature of from 60 to 200°C after the reaction with the olefinically or acetylenically unsaturated hydrocarbons.

**15.** Use of the selectively surface-hydrophilized silicone elastomers according to one or more of Claims 1 to 10 for the production of articles for the medical and medico-technical sector.

**16.** Articles for the medical and medico-technical sector produced using the selectively surface-hydrophilized silicone elastomers according to one or more of Claims 1 to 10.

**Revendications**

**1.** Élastomères silicones rendus de façon sélective hydrophiles en surface, dont les atomes de silicium se trouvant sur la surface se présentent à raison de 1 à 100 % sous forme d'unités de formule

$$[Z\ X\ Y\ SiO_{1/2}] \qquad (I)$$

dans laquelle

X représente un groupe alkyle en $C_1$-$C_{12}$ linéaire, ramifié ou cyclique, un groupe alkyle en $C_1$-$C_{20}$ substitué par des hétéroéléments ou un groupe aryle en $C_6$-$C_{10}$,

Z représente $O_{1/2}$ ou X, et

Y représente un groupe

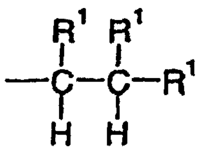 ou 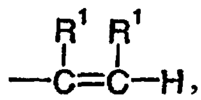

où

$R^1$ représente H, un groupe alkyle en $C_1$-$C_{12}$ linéaire, ramifié ou cyclique, un groupe alkyle en $C_1$-$C_{20}$ substitué par des hétéroéléments ou un groupe aryle en $C_6$-$C_{10}$, les restes $R^1$ pouvant être identiques ou différents à l'intérieur d'une molécule, à condition qu'au moins l'un des restes R' soit choisi parmi:

$R^1$ =$-CH_2OH$,
$-CH_2(OCH_2CH_2)_m(OCH_2CH[CH_3])_n(OCH_2CH[CH_2OH])_oOE$,
$-CH_2OC(O)CH_2CH_2COOH$,
$-CH_2OC(O)CH_2CH_2COO^-$,
$-CH_2OC(O)CH=CHCOOH$,
$-CH_2OC(O)CH=CHCOO^-$,
$-(CH_2)_pCH(OH)CH_2R^2$,
$-(CH_2)_pOCH_2CH(OH)CH_2R^2$,
$-(CH_2)_qR^3$,
$-(CH_2)_pO(CH_2)_pR^3$,

où
m = 0 à 100,
n = 0 à 60,
o = 0 à 60, et
$0 \leq m+n+o \leq 100$,
p = 1 à 10,
q = 0 à 5,

E représente un groupe $CH_3$, H, $C_4H_9$, t-$C_4H_9$,

$R^2$ =$-NH[(CH_2)_2NH]_sR^4$,
$-NH[(CH_2)_3]_sR^4$,
$-NHCH_2CH(CH_3)NHR^4$,
$-N[(CH_2)_2NHR^4]_2$,
$-N[(CH_2)_3NHR^4]_2$,

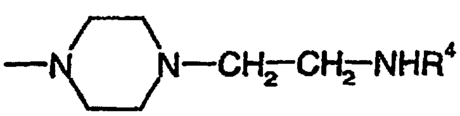

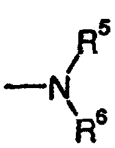

$R^3$ représente un groupe $-(CF_2)_tCF_3$ ou $-(CF_2)_tCF_2H$,

où
s = 0 à 10,
t = 0 à 9,

$R^4$ représente H ou un reste hydrocarboné en $C_1$-$C_{12}$ mono- ou polyhydroxylé linéaire et/ou cyclique,

$R^5$ représente H ou un groupe alkyle en $C_1$-$C_{12}$ ou hydroxyalkyle, et

$R^6$ représente un groupe alkyle en $C_1$-$C_{12}$, un reste hydrocarboné en $C_1$-$C_{12}$ mono- ou polyhydroxylé linéaire et/ou cyclique de formule $-CH_2CH_2OH$, $-CH_2CH_2CH_2OH$, $-CH_2CH_2CH_2CH_2OH$, $-CH_2CH(OH)CH_2OH$,

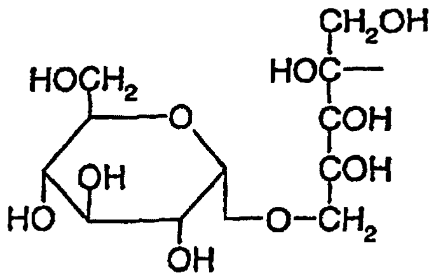 , 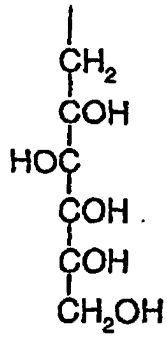 .

2. Élastomères silicones rendus de façon sélective hydrophiles en surface selon la revendication 1, **caractérisés en ce que** le reste $R^4$ représente des mono- ou disaccharides oxydés de formule

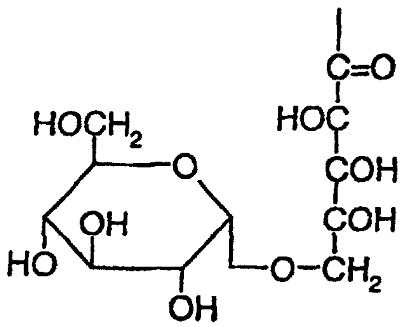 , 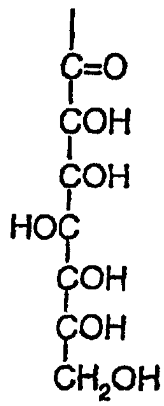 , 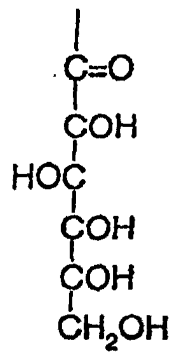 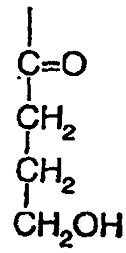

3. Élastomères silicones rendus de façon sélective hydrophiles en surface selon la revendication 1, **caractérisés en ce que** l'élastomère silicone est un polymère réticulé ayant une viscosité, mesurée à 20°C selon la norme DIN 53 015, 53 518 ou 53 019, de 2 Pa·s à 300 Pa·s, et est constitué d'unités de formule II

$$M_aSiO_{\frac{4-a}{a}} \quad (II)$$

dans laquelle
M est H, un groupe alkyle en $C_1$-$C_{12}$ linéaire, ramifié ou cyclique, un groupe alkyle en $C_1$-$C_{20}$ substitué par des hétéroéléments ou un groupe aryle en $C_6$-$C_{10}$, et
a = 0, 1, 2 et/ou 3.

4. Élastomères silicones rendus de façon sélective hydrophiles en surface selon la revendication 3, **caractérisés en ce que** le polymère (II) présente des unités T et/ou Q comme sites de ramification, et contient éventuellement des charges.

5. Élastomères silicones rendus de façon sélective hydrophiles en surface selon la revendication 3 ou 4, **caractérisés en ce que** le polymère de formule (II) présente des ponts $(CH_2)_w$ substitués par allyle en $C_1$-$C_3$, où w = 2 ou 3, entre deux atomes de silicium, qui résultent de la réaction de structures Si-H avec des composés vinyliques ou allyliques.

6. Élastomères silicones rendus de façon sélective hydrophiles en surface selon l'une ou plusieurs des revendications 1 à 5, **caractérisés en ce que** X est un groupe 1,1,1-trifluoropropyle.

7. Élastomères silicones rendus de façon sélective hydrophiles en surface selon l'une ou plusieurs des revendications

1, 3, 4, 5 ou 6, **caractérisés en ce qu'**ils présentent une couche d'oxyde entre la surface rendue hydrophile et la masse, le reste $R^1$ ayant la définition donnée dans la revendication 1.

**8.** Élastomères silicones rendus de façon sélective hydrophiles en surface selon la revendication 7, **caractérisés en ce que** le reste $R^4$ représente des mono- et disaccharides selon la revendication 2.

**9.** Élastomères silicones rendus de façon sélective hydrophiles en surface selon la revendication 7, **caractérisés en ce que** le reste $R^5$ est un reste $-CH_2CH_2OH$, $-CH_2CH_2CH_2OH$ et $-CH_2CH_2CH_2CH_2OH$.

**10.** Élastomères silicones rendus de façon sélective hydrophiles en surface selon la revendication 7, **caractérisés en ce que** le reste $R^6$ est un reste $-CH_2CH_2OH$, $-CH_2CH_2CH_2OH$ et $-CH_2CH_2CH_2CH_2OH$, ainsi que $-CH_2CH(OH)CH_2OH$,

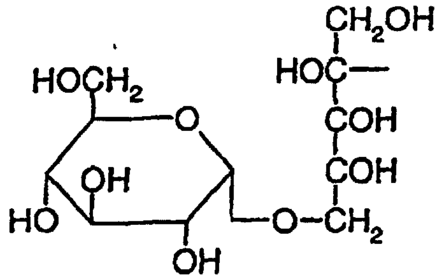
,
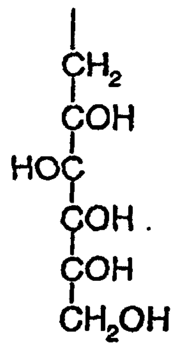
.

**11.** Procédé de préparation d'élastomères silicones rendus de façon sélective hydrophiles en surface selon la revendication 1, **caractérisé en ce que**, dans un élastomère silicone hydrophobe,

1. on transforme au moins une liaison Si-alkyle sur la surface a) en SiOH et/ou $SiO_2$ ou b) en SiH, puis
2.a) soit on fait réagir les groupes SiOH et/ou $SiO_2$ sur la surface avec un silane de formule

$$H_xSiB_{4-x}$$

dans laquelle B = Cl, Br, $OCH_3$ ou $OC_2H_5$, et
x = 1-3,
puis on fait réagir ce produit de réaction avec des composés oléfiniques ou acétyléniques fonctionnels en présence d'un catalyseur à base de platine,
soit on fait réagir les groupes SiOH et/ou $SiO_2$ sur la surface avec un silane de formule

$$Y_xSiB_{4-x}$$

dans laquelle B = Cl, Br, $OCH_3$ ou $OC_2H_5$, et
x = 1-3,
et Y a la définition donnée dans la revendication 1, et
2.b) on fait réagir les groupes SiH sur la surface avec des hydrocarbures à insaturation oléfinique ou acétylénique ayant la structure générale

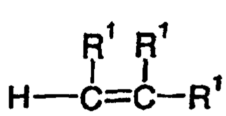
ou
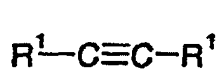

où les restes $R^1$ ont une définition correspondant à la définition de $R^1$ dans la revendication 1, et
3. au lieu de la transformation des groupes Si-alkyle en groupes SiH, on utilise directement un polysiloxane à fonctions SiH réticulé préparé par réaction d'un siloxane à fonctions SiH avec un polysiloxane contenant des doubles liaisons C=C en présence d'un catalyseur à base d'un métal de transition.

**12.** Procédé de préparation d'élastomères silicones rendus de façon sélective hydrophiles en surface selon la revendication 11, **caractérisé en ce que** les composés oléfiniques ou acétyléniques fonctionnels, avec lesquels on fait réagir le produit de la réaction des groupes SiOH et/ou $SiO_2$ avec le silane de formule $H_xSiB_{4-x}$, sont des polyéthers, des polyamines et/ou des polyamides contenant des groupes insaturés, x et B ayant la définition indiquée dans la revendication 11.

**13.** Procédé selon la revendication 11, **caractérisé en ce que** la transformation de la liaison Si-allyle en $SiO_2$ et/ou SiOH et SiH s'effectue par traitement au plasma.

**14.** Procédé selon la revendication 11, **caractérisé en ce que**, après la réaction avec les hydrocarbures à insaturation oléfinique ou acétyléniques, on amène le polysiloxane à fonctions SiH réticulé à une température de 60 à 200°C.

**15.** Utilisation d'élastomères silicones rendus de façon sélective hydrophiles en surface selon l'une ou plusieurs des revendications 1 à 10 pour la préparation d'articles destinés au domaine de la médecine ou de la technique médicale.

**16.** Articles destinés au domaine de la médecine ou de la technique médicale, qui ont été préparés à l'aide des élastomères silicones rendus de façon sélective hydrophiles en surface selon l'une ou plusieurs des revendications 1 à 10.